# EUROPEAN PATENT APPLICATION

(11) **EP 3 056 141 A1**
(43) Date of publication of application: **17.08.2016**
(21) Application number: 15460074.6
(22) Date of filing: 23.09.2015
(51) Int. Cl.: A61B 5/0205

(54) **A METHOD OF NON-INVASIVE MEASUREMENT OF BLOOD GLUCOSE AND OPTOELECTRONIC SYSTEM TO NON-INVASIVE MEASUREMENT OF BLOOD GLUCOSE**

(30) Priority: 10.02.2015 PL 41122315
(71) Applicant: Politechnika Gdanska, 80-233 Gdansk (PL)
(72) Inventor: Smulko, Janusz, 81-198 Suchy Dwór (PL); Gnyba, Marcin, 80-180 Borkowo (PL); Wróbel, Maciej, 82-200 Malbork (PL)

(57) **Abstract**

A method and system for non-invasive measurement of blood glucose involving the use of Raman spectra and measurements of pulse modulated content of red blood cells in the illuminated tissue and use of phase sensitive detection reducing the impact of noise from measurement system and differences in tissues between individuals. The optoelectronic system includes an excitation laser (LR), that emits radiation passing through the Raman probe (SR) and illuminating a tissue under test (TK). The Raman scattering radiation is collected by the Raman probe (SR) and recorded by the camera (KR) after having passed through the spectrometer (SP). Simultaneously, pulse is measured by pulse oximetry (PO) in similar tissue volume of which Raman scattering radiation is recorded. For both signals the phase sensitive detection is applied, performed by the control computer (KS), wherein multiplier and a low pass filter are implemented. Phase sensitive detection enables extraction of the signal proportional to blood glucose from the Raman spectrum, reducing the noise of the measuring system and effects derived from differences in the composition of illuminated tissue caused by the variability of individual.

## Description

The invention relates to a method and system for non-invasive measurement of blood glucose, which does not require violations of tissues, especially applicable in the diagnosis and treatment of diabetes especially for the purposes of self control.

Known method of detection and determination of concentration of chemical substances using Raman phenomenon consists is based use of laser excitation and measurement of radiation scattered on a sample by Raman phenomenon (Raman scattering). In the recorded Raman spectrum spectral lines appear. They are characteristic for the chemical bonds of the illuminated substance and their intensity is proportional to the concentration of the substance.

This method is used in research to evaluate the composition of the biological tissues by their irradiating by laser and analysis of recorded Raman scattering of radiation (eg. Barman, I., Dingari, N.C. Singh, G.P., Soares, J.S., Dasari, R.R., & Smulko, J.M. (2012). Investigation of Noise-induced instabilities in quantitative biological spectroscopy and its implications for noninvasive glucose monitoring. Analytical Chemistry, 84 (19), p.8149-8156). The method is very attractive, but due to differences in structure of tissue between the study of living organisms (eg. different thickness fat, water content, placed on turbidity, etc.) it requires calibration by using a reference method, which is invasive.

Moreover, the intensity of the Raman scattering signal is less than 0.5% of the energy of the whole acquired spectrum. It is necessary to find a solution enabling the extraction of Raman signals coming from blood components, particularly glucose contained in the blood and the separation of the signals coming from the surrounding tissues.

One of the proposed approaches is a mechanical modulation of the irradiated tissue by applying gentle pressure (eg. Chaiken, J., Goodisman, J. Deng, B., Bussjager, RJ, &Shaheen, G. (2009). Simultaneous, noninvasive observation of elastic scattering,fluorescence and inelastic scattering as a monitor of blood flow and hematocrit inhuman fingertip capillary beds, Journal of Biomedical Optics, 14 (5), 050505-050,505th), which is also described in US Patent US6377828. This solution assumes that compression of tissues changes its content of blood, which is marked by the number of red blood cells in the illuminated tissue area with subsequent acquisition of a signal dependent on the magnitude of tissue compression, even though the content of red blood cells is less than about 1% of the volume of illuminated tissue. Such effect caused by compression is possible, because the scattering coefficient (µₛ = 300 cm⁻¹) of red blood cells is more than twenty times greater than that of the other components of illuminated tissues, such as plasma (µₛ = 0.6 cm⁻¹) or solid tissue (e.g. skin: µₛ = 12 cm⁻¹). Thus, a slight change in the content of red blood cells in the illuminated tissues significantly affects the changes in the intensity of spectral components of registered Raman scattering radiation. Such solution, however, still has some drawbacks, and the Raman scattering signal is still very weak. In addition, the measurement results are still affected by the individual characteristics of the irradiated tissue causing the need for calibration using a reference method. Application of multiple gentle pressings of the illuminated tissue (i.e. the modulation of the content of red blood cells) should allow for the use of synchronous (phase sensitive) detection subsequently improving the signal (Raman scattering radiation) to noise ratio, as well as reduce the influence of individual characteristics of tested tissues. Practically, one should expect only a slight improvement, since the effectiveness of synchronous detection is limited by the lack of knowledge of the waveform of the signal which is modulating the changes in the tissues. Such modulating signal should be proportional to changes in blood content of the illuminated tissue area, and not to the intensity of applied pressure (e.g. changes in the forces of pressing on tissue over the course of time, while the Raman scattering signal is recorded).

In view of these disadvantages it is proposed herein another method of elimination of the impact of differences between individuals through the identification of reference signal which depends on blood content (the number of red blood cells) in the examined tissues. The invention comprises a method of operation and construction of the measuring system for determination of blood glucose without affecting the continuity of tissues (i.e. non-invasive) using synchronous detection method using measurements of Raman spectra and pulse oximetry measurements, where the measurement of pulse oximetry is a source of synchronization signal for Raman measurement.

A method of non-invasive measurement of blood glucose involving: illumination of a fragment of a studied tissue with laser radiation, collecting feedback Raman scattering signal by a Raman probe, splitting this light by a spectrograph into individual spectral components and recording of those features by a camera. Such invention is specific in the sense, that at the same time, a pulse oximeter measures a pulse and records temporal changes in the amount of blood in the tissues of the studied tissue area similar in volume to the area from which Raman scattering radiation is recorded simultaneously.

The resulting signal, corresponding to temporal changes in pulse, is used for the synchronous detection of the Raman signal implemented by the control computer. Therein, through the use of the implemented multiplier circuits, signals from the camera, pulse oximeter, and a low-pass filter which eliminates signals of pulse frequency and doubled pulse frequency, a specific component of a Raman scattering signal is selected, wherein this component of the signal is induced by the Raman scattering on blood, whereas other components of a Raman signal are caused by scattering on the other components of the tested tissue. Component caused by scattering on blood is proportional to the amount of glucose in the blood.

Optoelectronic system for non-invasive measurement of blood glucose comprising an excitation laser coupled to the Raman probe, which is connected to a spectrometer and a camera. Such invention is specific in the sense, that the camera is connected to a control computer executing synchronous detection of the Raman signal with the use of the signal corresponding to the temporal changes of pulse originating from a pulse oximeter.

The use of the pulse oximeter enables to obtain a signal proportional to the content of red blood cells modulated by the pulse. This, in turn, allows one to synchronize the Raman measurement signal, and the use of synchronized detection algorithm, (described in the literature, e.g. Kotarski, M., & Smulko, J. (2009, September) "Assessment of synchronic detection at low frequencies through DSP-based board and PC sound card" In the XIX IMEKO World Congress, pp. 960-963), allowing partial separation (differentiation) in the time-domain the signals from the blood and signals from the components originating from the surrounding tissues, because in the different phases of the pulse cycle, the content of blood in the illuminated area is different. Moreover, it will enable reducing the noise in the recorded Raman spectra as a result of averaging, and reduce the effect of Raman scattering phenomena from surrounding tissue with properties different for each individual.

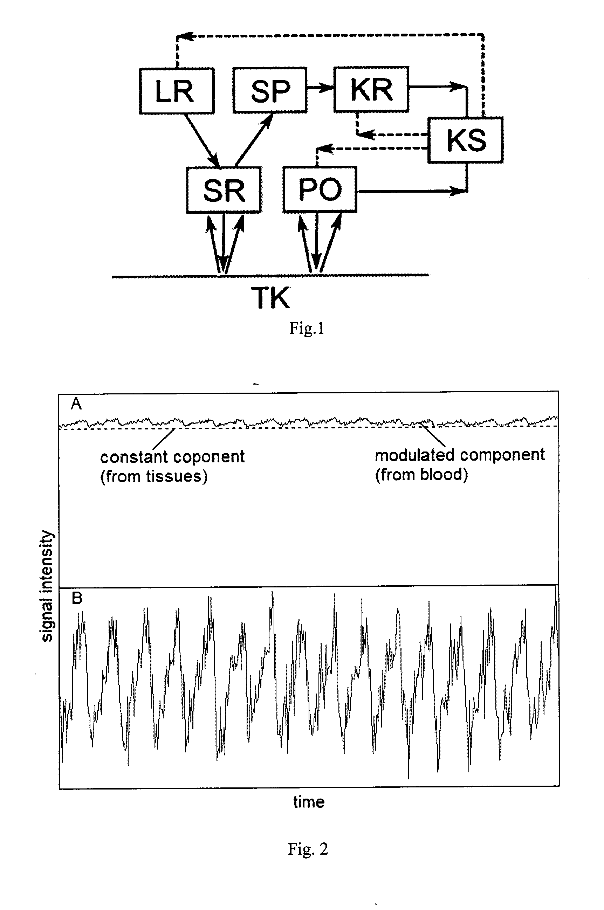

The invention is further explained with reference to the accompanying drawings, where Fig. 1 shows a block diagram of a system for non-invasive measurement of blood glucose, wherein arrows with solid lines indicate the flow directions of measurement signals, and arrows of dotted lines - the measurement control signals, and Fig. 2 shows a plot of temporal Raman signals for a selected exemplary wavelengths, coming from tissue TK, comprising the component derived from blood, modulated by the signal of pulse and non-modulated component derived from the surrounding tissue caused by inter-individual variability; and the signal recorded by pulse oximeter.

In the presented invention, the excitation radiation from the laser LR is filtered and focused by the Raman probe SR and subsequently it illuminates the tested tissue CT. The probe SR focuses part of Raman scattered signal and transmits it back to the spectrometer SP. Raman scattering radiation is there spectrally separated, then recorded by a camera and recorded as the Raman spectrum in the control computer KS. Time waveforms of main signals are shown in Fig. 2. A Raman signal A includes an AC (variable) component of blood, which is modulated with frequency of pulse as well as a DC (static) component derived primarily from the surrounding tissues, which must be eliminated, because it is highly dependent on individual characteristics.

Measurement of the Raman spectrum is performed by a series of short duration measurements repeated at a frequency of at least twice as the pulse, which were subsequently subjected to phase sensitive detection operations at the computer. This allows restoration of time waveform as a variable component that contains useful information about the level of glucose in the blood.

Simultaneously, a signal from the pulse oximeter PO is recorded, informing about the pulse and changes in oxygen concentration, and indirectly - about changes in blood concentration in the area of tissue TK, close to the area where the laser radiation is scattered. By use of the phase sensitive detection, signal proportional to the change of blood content in the illuminated tissue can be obtained from the Raman spectrum. Other components are suppressed due to averaging.

Fig. 2 shows waveforms of signals: A - Raman signal for the selected exemplifying wavelength generated in the tissue TK, comprising the component derived from blood modulated by pulse signal and the DC component derived from the surrounding tissue influenced by inter-individual variability; B - signal recorded by the pulse oximeter.

The pulse oximeter PO uses for its work a phenomenon of absorption of other wavelengths than spectral range used for Raman measurements. Compared to existing solutions using tissue modulation it offers better efficiency of the phase sensitive detection, because recorded reference signal is proportional to the content of blood in illuminated tissues and thereby the component assigned to scattering of red blood cells can be specified in the registered Raman scattering radiation, while component due to scattering in other tissues would be eliminated. The control computer KS performs phase sensitive detection in two steps. In the first step Raman signal is multi-channel multiplied by a variable signal B from the pulse oximeter PO. Since these waveforms are modulated by the same factor, on the output two components can be obtained: DC (static) and random component, representative for measurement errors and the effects of finite averaging time. In the next step - low-pass filtering - the random component is filtered out, and only the constant signal assigned to the Raman phenomenon occurring in the blood is given to the output.

The camera KR and the pulse oximeter PO are connected to the control computer KS, which initializes the measurement procedures and performs the phase sensitive detection. The control computer KS works as the real-time multiplier of the signals from the camera KR and pulse oximeter PO. Moreover, the KS works as the low-pass filter eliminating signals having pulse frequency and doubled pulse frequency. The signals obtained from the camera KR come from the connected spectrometer SP, which is subsequently connected to the Raman probe SR. Raman probe SR is connected to a source of laser radiation LR.

## Claims

1. A method for non-invasive measurement of blood glucose based on illumination of the examined fragment of tissue by the laser radiation, collection of Raman backscattered radiation by the probe Raman, separation of the spectral components by the spectrometer and their recording by the camera **characterized by** simultaneously carried out the measurement of the pulse by a pulse oximeter (PO) and recording of temporal changes in the amount of blood in studied tissue area (TK) having similar volume to the area from which Raman scattering radiation is recorded, then the obtained signal corresponding to pulse-time waveform is used for phase sensitive detection of Raman scattering signal which is carried out by control computer (KS) that performs, in the real time, multiplication of the signals from the camera (KR), pulse oximeter (PO) and low-pass filter eliminating signals having pulse frequency and doubled pulse frequency, and separation a component of Raman signal assigned to light scattering by blood components, which is proportional to the glucose content in the blood, from the other components of the test body (TK) caused by Raman scattering.

2. The optoelectronic system for non-invasive measurement of blood glucose comprises of a laser emitting an excitation radiation and coupled to the Raman probe, which is connected to a spectrometer and to a camera **characterized by** that the camera (KR) is connected to the control computer (KS), which implements phase sensitive detection of the Raman signal through the use of the signal corresponding to pulse-time waveform coming from the computer (KS) controlling the pulse oximeter (PO).
